# EUROPEAN PATENT APPLICATION

(11) **EP 3 712 847 A1**
(43) Date of publication of application: **23.09.2020**
(21) Application number: 20164664.3
(22) Date of filing: 20.03.2020
(51) Int. Cl.: G06T 7/00, G06T 7/10, G06T 7/73, G06T 11/00

(54) **CATHETER TIP DETECTION IN FLUOROSCOPIC VIDEO USING DEEP LEARNING**

(30) Priority: 21.03.2019 US 201962821696 P; 23.05.2019 US 201962852092 P; 26.11.2019 US 201962940686 P; 23.02.2020 US 202016798378
(71) Applicant: Covidien LP, Mansfield, MA 02048 (US)
(72) Inventor: ALEXANDRONI, Guy, 3522420 Haifa (IL)
(74) Representative: Maschio, Antonio

(57) **Abstract**

A system and method of pose estimation to enable generation of high-quality 3D reconstruction volumes and fluoroscopic computed tomography images for the identification of small and ground glass lesions.

## Description

### CLAIM OF PRIORITY

This application claims priority to U.S. Provisional Application Serial No. 62/940,686 titled CATHETER TIP DETECTION IN FLUOROSCOPIC VIDEO USING DEEP LEARNING and filed November 26, 2019 and U.S. Provisional Application Serial No. 62/852,092 titled CATHETER TIP DETECTION IN FLUOROSCOPIC VIDEO USING DEEP LEARNING and filed May 23, 2019 and U.S. Provisional Application Serial No. 62/821,696 titled CATHETER TIP DETECTION IN FLUOROSCOPIC VIDEO USING DEEP LEARNING and filed, March 21, 2019, the entire contents of each are incorporated herein by reference.

### FIELD

This disclosure relates to the field of imaging, and particularly to the estimation of a pose of an imaging device to improve the clarity of fluoroscopic computed tomography images derived therefrom.

### BACKGROUND

A fluoroscopic imaging device is commonly located in the operating room during navigation procedures. The standard fluoroscopic imaging device may be used by a clinician, for example, to visualize and confirm the placement of a medical device after it has been navigated to a desired location. However, although standard fluoroscopic images display highly dense objects such as metal tools and bones as well as large soft-tissue objects such as the heart, the fluoroscopic images have difficulty resolving small soft-tissue objects of interest such as lesions. Furthermore, the fluoroscope image is only a two-dimensional projection, while in order to accurately and safely navigate within the body, a volumetric imaging is required.

Pose estimation of the fluoroscopic device is a step employed as part of a three-dimension (3D) reconstruction process, where a 3D volume is generated from the two-dimensional (2D) fluoroscopic images resulting in a Fluoroscopic Computed Tomography (FCT) image set. In addition, the pose estimation can assist in registration between different imaging modalities (e.g., pre-operative Computed Tomography (CT) images). Prior art methods of pose estimation, while effective, nonetheless suffer from a lack of robustness or have resulted in slower than desired processing of the images.

Therefore, there is a need for a method and system, which can provide a fast, accurate and robust pose estimation of a fluoroscopic imaging device and 3D reconstruction of the images acquired by a standard fluoroscopic imaging device.

### SUMMARY

One aspect of the disclosure is directed to a method for enhancing fluoroscopic computed tomography images including: acquiring a plurality of fluoroscopic images, determining an initial pose estimation of a fluoroscopic imaging device for each of the plurality of fluoroscopic images, receiving an indication of a catheter tip in at least one of the fluoroscopic images, projecting a position of the catheter tip in the remaining fluoroscopic images, analyzing the images with a model to identify a location of the catheter tip in the fluoroscopic images, updating the initial pose estimation based on the location of the catheter tip identified by the model, and generating a three-dimensional (3D) reconstruction of the plurality of fluoroscopic images utilizing the updated pose estimation. Other embodiments of this aspect include corresponding computer systems, apparatus, and computer programs recorded on one or more computer storage devices, each configured to perform the actions of the methods and systems described herein.

A further aspect of the disclosure may include one or more of the following features. The method may include displaying a fluoroscopic computed tomography image derived from the 3D reconstruction. The method may include cropping the plurality of fluoroscopic images on which the position of the catheter tip was projected to define a region of interest. The method may include determining a confidence estimate for the detection of the catheter tip in each cropped fluoroscopic image. The method may include identifying two additional cropped fluoroscopic images located on either side, in the order in which the plurality of fluoroscopic images was acquired, of a cropped fluoroscopic image having a confidence estimate below a determined threshold. The method where the two additional cropped fluoroscopic images have a confidence estimate higher than the determined threshold. The method may include interpolating a position of the tip of the catheter in the cropped fluoroscopic image with a confidence estimate lower than the determined threshold from the position of the tip of the catheter in the two cropped fluoroscopic images with a confidence estimate higher than the determined threshold. The method where the received indications of the catheter tip is automatically generated. The method where the initial estimate of pose includes generating a probability map for each of the plurality of fluoroscopic images indicating a probability that each pixel of the fluoroscopic image belongs to a projection of a marker. The method further includes generating candidates for projection of the marker on the fluoroscopic image. The method may include identifying the candidate with the highest probability of projection of the marker being the projection of the marker on the image based on the probability map. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

A further aspect of the disclosure is directed a system for enhancing fluoroscopic computed tomography images including: a computing device in communication with a fluoroscopic imaging device and including a processor and a memory, the memory configured to store a plurality of fluoroscopic images and an application that when executed by the processor causes the processor to execute the steps of: determining an initial pose estimation of a fluoroscopic imaging device for each of the plurality of fluoroscopic images; receiving an indication of a catheter tip in at least one of the fluoroscopic images; projecting a position of the catheter tip in the remaining fluoroscopic images; and cropping the fluoroscopic images with the projected position of catheter tip to define a set of frames; a model derived from a neural network in communication with the memory and configured to analyze the frames to identify a location of the catheter tip in the frames; the memory receiving the identified locations of the tip of the catheter in the frames from the model and the processor executing steps of the application of updating the initial pose estimation based on the location of the catheter tip identified by the neural network; and generating a three-dimensional (3D) reconstruction of the plurality of fluoroscopic images utilizing the updated pose estimation. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The system where the processor further executes steps of the application of displaying a fluoroscopic computed tomography image derived from the 3D reconstruction. The system where the processor further executes steps of the application of generating a confidence estimate of detection of the tip of the catheter for each cropped fluoroscopic image. The system where the processor further executes steps of the application of identifying two additional cropped fluoroscopic images located on either side, in the order in which the plurality of fluoroscopic images was acquired, of a cropped fluoroscopic image having a confidence estimate below a determined threshold. The system where the two additional cropped fluoroscopic images have a confidence estimate higher than the determined threshold. The system where the processor further executes steps of the application of interpolating a position of the tip of the catheter in the cropped fluoroscopic image with a confidence estimate lower than the determined threshold from the position of the tip of the catheter in the two cropped fluoroscopic images with a confidence estimate higher than the determined threshold. The system where the initial estimate of pose includes generating a probability map for each of the plurality of fluoroscopic images indicating a probability that each pixel of the fluoroscopic image belongs to a projection of a marker. The system further includes generating candidates for projection of the marker on the fluoroscopic image and identifying the candidate with the highest probability of projection of the marker being the projection of the marker on the image based on the probability map.

A further aspect of the disclosure is directed to a method for enhancing fluoroscopic computed tomography images including: acquiring a plurality of fluoroscopic images, determining an initial pose estimation of a fluoroscopic imaging device for each of the plurality of fluoroscopic images, receiving an indication of a catheter tip in at least two of the fluoroscopic images, projecting a position of the catheter tip in the remaining fluoroscopic images, cropping the plurality of fluoroscopic images on which the position of the catheter tip was projected to generate a plurality of frames, analyzing each frame as a main frame to determine a location of the catheter tip in the main frame, comparing the position of the catheter tip in each main frame to a position of the catheter tip in at least two additional frames to confirm the determined location of the catheter tip in each main frame, updating the initial pose estimation based on the confirmed location in each main frame, generating a three-dimensional (3D) reconstruction of the plurality of fluoroscopic images utilizing the updated pose estimation, and displaying a fluoroscopic computed tomography image derived from the 3D reconstruction. Other embodiments of this aspect include corresponding computer systems, apparatus, and computer programs recorded on one or more computer storage devices, each configured to perform the actions of the methods and systems described herein.

### BRIEF DESCRIPTION OF THE DRAWINGS

Various exemplary embodiments are illustrated in the accompanying figures. It will be appreciated that for simplicity and clarity of the illustration, elements shown in the figures referenced below are not necessarily drawn to scale. Also, where considered appropriate, reference numerals may be repeated among the figures to indicate like, corresponding or analogous elements. The figures are listed below.
FIG. 1 is a schematic diagram of a system configured for use with the method of the disclosure. a flow chart of a method for estimating the pose of an imaging device by utilizing a structure of markers in accordance with the disclosure;
FIG. 2 is a schematic illustration of a two-dimensional grid structure of sphere markers in accordance with the disclosure;
FIG. 3 is a flow chart of a method of the disclosure;
FIG. 4 shows an exemplary image captured by a fluoroscopic device of the disclosure;
FIG 5 is a probability map generated for the image of FIG. 4;
FIG 6A-6C show different exemplary candidates for the projection of the 2D grid structure of sphere markers of FIG. 2 on the image of FIG. 4 overlaid on the probability map of FIG. 5;
FIG. 7 depicts a user interface of the disclosure for marking a tip of a catheter in fluoroscopic images in accordance with the disclosure;
FIG. 8 depicts the results of a neural network identifying the location of the tip of a catheter in cropped frames in accordance with the disclosure;
FIG. 9 depicts two fluoroscopic computed tomography images, one using known pose estimation techniques and one using the pose estimation techniques of the disclosure;
FIG. 10 is a schematic drawing of computing device in accordance with the disclosure; and
FIG. 11 is a user-interface of an application for navigation of the airways in accordance with the disclosure.

### DETAILED DESCRIPTION

The disclosure is directed to a system and method of pose estimation that overcomes the drawbacks of the prior art pose estimation techniques and generates high quality 3D reconstruction volumes and FCT image sets. The higher quality 3D reconstruction and FCT image sets achieve greater resolution of the soft tissues. The greater resolution of the soft tissues enables identification of smaller and ground glass lesions in the soft tissues than achievable using prior techniques.

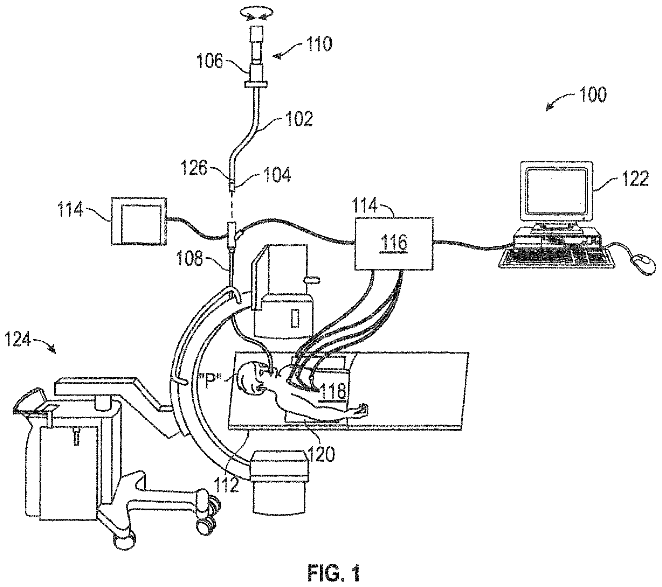

FIG. 1 is a perspective view of an exemplary system for navigation of a medical device, e.g., a biopsy or treatment tool, to a target via airways of the lungs. One aspect of the system 100 is a software application for reviewing computed tomography (CT) image data that has been acquired separately from system 100. The review of the CT image data allows a user to identify one or more targets and plan a pathway to an identified target. This is typically referred to as a planning phase. Another aspect of the software application is a navigation phase which allows a user to navigate a catheter or other tool to a target (navigation phase) using a user interface and confirm placement of the catheter or a tool relative to the target. The target is typically tissue of interest for biopsy or treatment that was identified during the planning phase by review of the CT image data. Following navigation, a medical device, such as a biopsy tool or treatment tool, may be inserted into the catheter to obtain a tissue sample from the tissue located at, or proximate to, the target or to treat such tissue. The treatment tool may be selected to achieve microwave ablation, radio-frequency ablation, cryogenic ablation, chemical ablation, or other treatment mechanism of the target as preferred by the clinician.

One aspect of FIG. 1 is a catheter guide assembly 102 including a sensor 104 at a distal end. The catheter guide assembly 102 includes a catheter 106. In practice, catheter 106 is inserted into a bronchoscope 108 for access to a luminal network of the patient P. Specifically, catheter 106 of catheter guide assembly 102 may be inserted into a working channel of bronchoscope 108 for navigation through a patient's luminal network. If configured for electromagnetic navigation (EMN) (as described below), a locatable guide (LG) 110, which may include the sensor 104 such as an electromagnetic (EM) sensor may be inserted into catheter 106 and locked into position such that sensor 104 extends a desired distance beyond the distal tip of catheter 106. However, it should be noted that the sensor 104 may be incorporated into one or more of the bronchoscope 108, catheter 106, or a biopsy or treatment tool, without departing from the scope of the disclosure.

If the catheter 106 is inserted into the bronchoscope 108, the distal end of the catheter 106 and LG 110 both extend beyond the distal end of the bronchoscope 108. The position or location and orientation of sensor 104 and thus the distal portion of LG 110, within an electromagnetic field can be derived based on location data in the form of currents produced by the presence of the EM sensors in a magnetic field, or by other means described herein. Though the use of EM sensors and EMN are not required as part of this disclosure, their use may further augment the utility of the disclosure in endoluminal navigation (e.g., navigation of the lungs). As the bronchoscope 108, catheter 106, LG 110 or other tool could be used interchangeably or in combination herein, the term catheter will be used here to refer to one or more of these elements. Further, as an alternative to the use of EM sensors, flex sensors such as fiber Bragg sensors, ultrasound sensors, accelerometers, and others may be used in conjunction with the present disclosure to provide outputs to the tracking system 114 for determination of the position of a catheter including without limitation the bronchoscope 108, catheter 106, LG 110, or biopsy or treatment tools, without departing from the scope of the present disclosure.

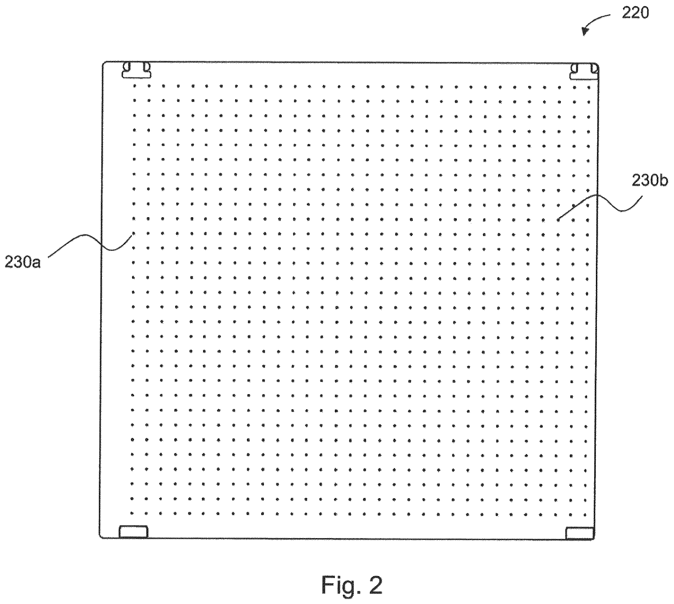

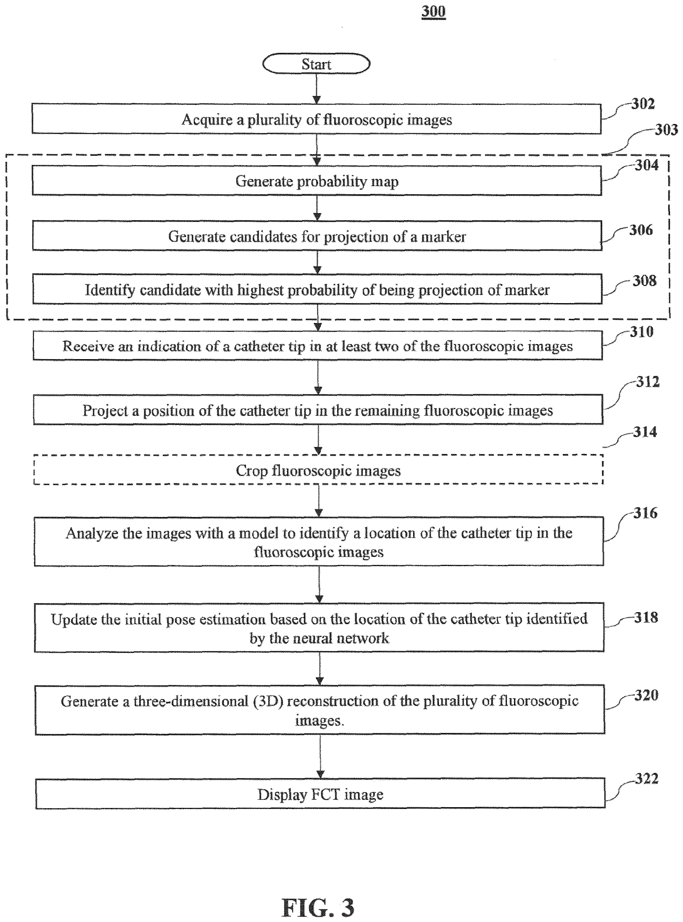

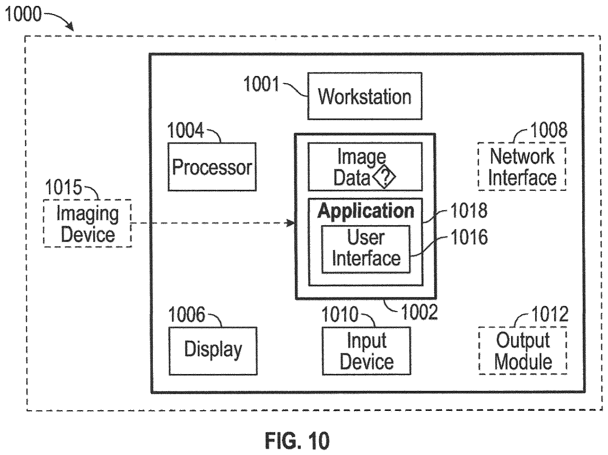

System 100 may generally include an operating table 112 configured to support a patient P, a bronchoscope 108 configured for insertion through patient P's mouth into patient P's airways; monitoring equipment 114 coupled to bronchoscope 108 (e.g., a video display, for displaying the video images received from the video imaging system of bronchoscope 108). If configured for EMN, system 100 may include a locating or tracking system 114 and a locating module 116, a plurality of reference EM sensors 118 and a transmitter mat 120 including a plurality of radio-opaque or partially radio-opaque markers 121 (FIG. 2). Though shown in FIG. 2 as a repeating pattern of markers 121, other patterns, including three dimensional markers at different relative depths in the transmitter mat 120, or a non-repeating pattern may be employed without departing from the scope of the present disclosure. Also included is a computing device 122 including software and/or hardware used to facilitate identification of a target, pathway planning to the target, navigation of a medical device to the target, and/or confirmation and/or determination of placement of catheter 106, or a suitable device therethrough, relative to the target. Computing device 122 may be similar to workstation 1001 of FIG. 10 and may be configured to execute the methods of the disclosure including the methods of FIG. 3. Computing device 122 may be any suitable computing device including a processor and storage medium, wherein the processor is capable of executing instructions stored on the storage medium as one or more applications. Computing device 122 may further include a database configured to store patient data, CT data sets including CT images, fluoroscopic data sets including fluoroscopic images and video, fluoroscopic 3D reconstruction, navigation plans, and any other such data. Although not explicitly illustrated, computing device 122 may include inputs, or may otherwise be configured to receive, CT data sets, fluoroscopic images/video and other data described herein. Additionally, computing device 122 includes a display configured to display graphical user interfaces. Computing device 122 may be connected to one or more networks through which one or more databases may be accessed. Further details of the computing device are described in connection with FIG. 10, below.

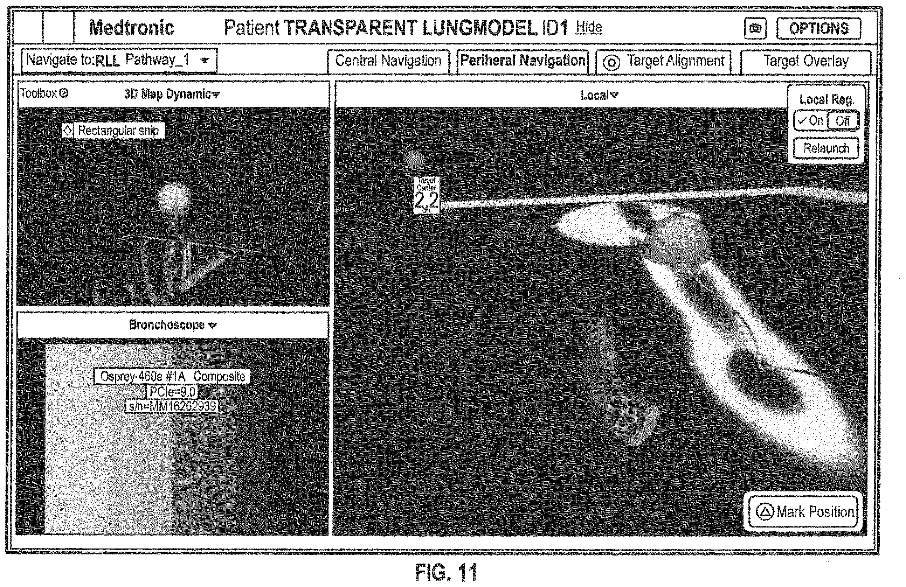

With respect to the planning phase, computing device 122 utilizes previously acquired CT image data for generating and viewing a three-dimensional model or rendering of patient P's airways, enables the identification of a target on the three-dimensional model (automatically, semi-automatically, or manually), and allows for determining a pathway through patient P's airways to tissue located at and around the target. More specifically, CT images and CT image data sets acquired from CT scans are processed and assembled into a three-dimensional CT volume, which is then utilized to generate a three-dimensional model of patient P's airways. The three-dimensional model may be displayed on a display associated with computing device 122, or in any other suitable fashion. An example of such a user interface can be seen in FIG. 11. Using computing device 122, various views of the three-dimensional model or enhanced two-dimensional images generated from the three-dimensional model are presented. The enhanced two-dimensional images may possess some three-dimensional capabilities because they are generated from three-dimensional data. The three-dimensional model may be manipulated to facilitate identification of target on the three-dimensional model or two-dimensional images, and selection of a suitable pathway through patient P's airways to access tissue located at the target can be made. Once selected, the pathway plan, three-dimensional model, and images derived therefrom, can be saved and exported to a navigation system for use during the navigation phase(s).

As noted above a fluoroscopic imaging device 124 capable of acquiring fluoroscopic or x-ray images or video of the patient P (fluoroscopic image data sets) is also included in system 100. The images, sequence of images, or video captured by fluoroscopic imaging device 124 may be stored within fluoroscopic imaging device 124 or transmitted to computing device 122 for storage, processing, and display. Additionally, fluoroscopic imaging device 124 may move relative to the patient P so that images may be acquired from different angles or perspectives relative to patient P to create a sequence of fluoroscopic images, such as a fluoroscopic video. The pose of fluoroscopic imaging device 124 relative to patient P and while capturing the images may be estimated using the markers 121 and pose estimation and image processing techniques described hereinbelow.

The markers 121 may be incorporated into the transmitter mat 120, incorporated into the operating table 112, or otherwise incorporated into another appliance placed on or near the operating table 112 so that they can be seen in the fluoroscopic images. The markers 121 are generally positioned under patient P and between patient P and a radiation source or a sensing unit of fluoroscopic imaging device 124. Fluoroscopic imaging device 124 may include a single imaging device or more than one imaging device.

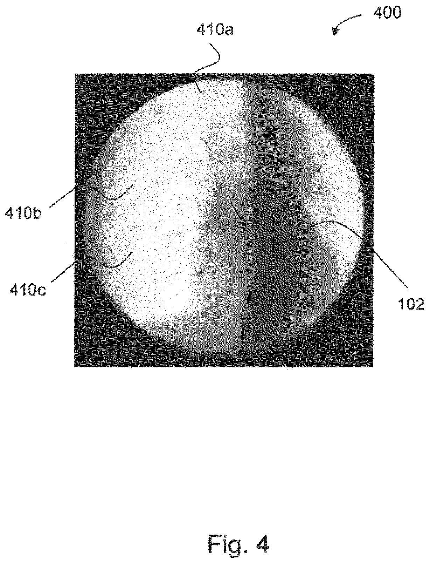

FIG. 3 is a flow chart for pose estimation and fluoroscopic computed tomography images in accordance with the present disclosure. As part of the procedure a catheter 106 is navigated to a desired location in the patient "P." This may be done by following the pathway plan and the EM system described above or under bronchoscopic imaging or under fluoroscopic imaging using fluoroscopic imaging device 124. Having navigated the catheter 106 to a desired location, a fluoroscopic sweep can be acquired at step 302. This fluoroscopic sweep acquires a plurality of 2D fluoroscopic images at different angles as the fluoroscopic imaging device 124 rotates about the patient "P." Each 2D image acquired by the fluoroscopic imaging device 124 includes the markers 121 as depicted in FIG. 4.

After acquiring the fluoroscopic images, an initial pose estimation process 303, comprised of steps 304-308, as described below. The computing device undertakes a pose estimation process for each of the 2D images acquired during the fluoroscopic sweep 302. The initial pose estimation 303 starts with step of generating a probability map at step 304. The probability map indicates the probability that a pixel of the image belongs to the projection of a marker 121 of the transmitter mat 120.

FIG. 2 is a schematic illustration of a two-dimensional (2D) grid structure of sphere markers 220 in accordance with the disclosure. FIG. 4 is an exemplary image 400 captured by a fluoroscopic imaging device 124 of a patient in which the 2D grid structure of markers 121 are visible. The 2D grid structure of sphere markers 220 includes a plurality of sphere-shaped markers, such as sphere markers 230a and 230b, arranged in a two-dimensional grid pattern. Image 400 includes a projection of a portion of 2D grid structure of sphere markers 220 and a projection of a catheter 106. The projection of 2D grid structure of sphere markers 220 on image 400 includes projections of the sphere markers, such as sphere marker projections 410a, 410b and 410c. A catheter 106 is also observable in the image.

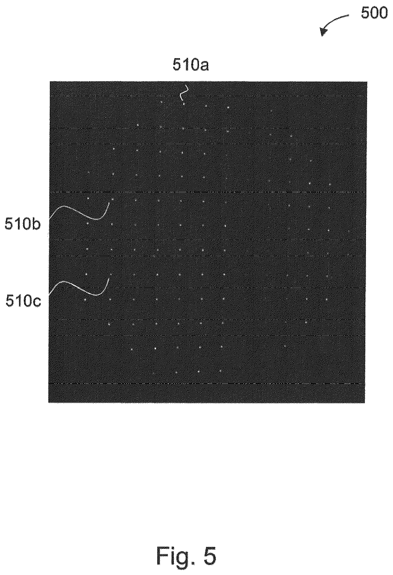

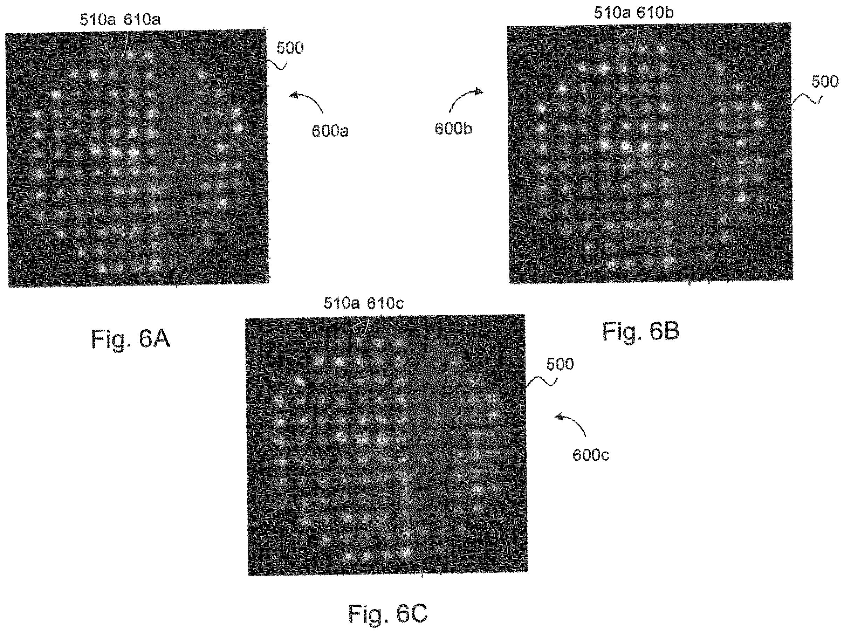

The probability map may be generated, for example, by feeding the image into a simple marker detector, such as a Harris corner detector, which outputs a new image of smooth densities, corresponding to the probability of each pixel to belong to a marker. Reference is now made to FIG. 5, which is a probability map 500 generated for image 400 of FIG. 4. Probability map 500 includes pixels or densities, such as densities 510a, 510b and 510c, which correspond accordingly to markers 410a, 410b and 410c. In some embodiments, the probability map may be downscaled (i.e., reduced in size) in order to simplify the required computations. It should be noted that probability maps 400, as shown in FIGs 6A-6C are downscaled by four (e.g., 1/4^{th} of the entire image).

In a step 306, different candidates may be generated for the projection of the structure of markers on the image. The different candidates may be generated by virtually positioning the imaging device in a range of different possible poses. By "possible poses" of the fluoroscopic imaging device 124, it is meant three-dimensional positions and orientations of the fluoroscopic imaging device 124. In some embodiments, such a range may be limited according to the geometrical structure and/or degrees of freedom of the imaging device. For each such possible pose, a virtual projection of at least a portion of the markers 121 is generated, as if the fluoroscopic imaging device 124 actually captured an image of the structure of markers 121 while positioned at that pose.

At step 308, the candidate having the highest probability of being the projection of the structure of markers 121 on the image is identified based on the image probability map. Each candidate, i.e., a virtual projection of the structure of markers, may be overlaid or associated to the probability map. A probability score may be then determined or associated with each marker projection of the candidate. In some embodiments, the probability score may be positive or negative, i.e., there may be a cost in case virtual markers projections falls within pixels of low probability. The probability scores of all of the markers projections of a candidate may be then summed and a total probability score may be determined for each candidate. For example, if the structure of markers is a two-dimensional grid, then the projection will have a grid form. Each point of the projection grid would lie on at least one pixel of the probability map. A 2D grid candidate will receive the highest probability score if its points lie on the highest density pixels, that is, if its points lie on projections of the centres of the markers on the image. The candidate having the highest probability score may be determined as the candidate which has the highest probability of being the projection of the structure of markers on the image. The pose of the imaging device while capturing the image may be then estimated based on the virtual pose of the imaging device used to generate the identified candidate.

Steps 304-308, above described one possible pose estimation process 303, however, those of skill in the art will recognize that other methods and processes of initial pose estimation may be undertaken without departing from the scope of the disclosure.

Reference is now made to FIGs 6A-6C, which show different exemplary candidates 600a-c for the projection of 2D grid structure of sphere markers 220 of FIG. 2 on image 400 of FIG. 4 overlaid on probability map 500 of FIG. 5. Candidates 600a, 600b and 600c are indicated as a grid of plus signs ("+"), while each such sign indicates the center of a projection of a marker. Candidates 600a, 600b and 600c are virtual projections of 2D grid structure of sphere markers 220, as if the fluoroscopic imaging device 124 used to capture image 400 is located at three different poses associated correspondingly with these projections. Candidate 600a was generated as if the fluoroscopic imaging device is located at: position [0, -50, 0], angle: -20 degrees. Candidate 600b was generated as if the fluoroscopic imaging device is located at: position [0, -10, 0], angle: -20 degrees. Candidate 600c was generated as if the fluoroscopic imaging device is located at: position [7.5, -40, 11.25], angle: -25 degrees. The above-mentioned coordinates are with respect to 2D grid structure of sphere markers 220. Densities 510a of probability map 500 are indicated in FIGs. 6A-6C. Plus signs 610a, 610b and 610c are the centers of the marker projections of candidates 600a, 600b and 600c correspondingly, which are the ones closest to densities 510a. One can see that plus sign 610c is the sign which best fits densities 510a and therefore would receive the highest probability score among signs 610a, 610b and 610c of candidates 600a, 600b and 600c correspondingly. One can further see that accordingly, candidate 600c would receive the highest probability score since its marker projections best fit probability map 400. Thus, among these three exemplary candidates, 600a, 600b and 600c, candidate 500c would be identified as the candidate with the highest probability of being the projection of 2D grid structure of sphere markers 220 on image 400.

As noted above, the pose estimation process 303 is undertaken for every image in the fluoroscopic sweep undertaken at step 302. The result of the processing is a determination of the pose of the fluoroscopic imaging device 124 for each image acquired. While this data can be used to generate the 3D reconstruction and where desired to register the 3D reconstruction to a 3D model generated from a pre-operative CT scan, further refinements can be undertaken to achieve superior results.

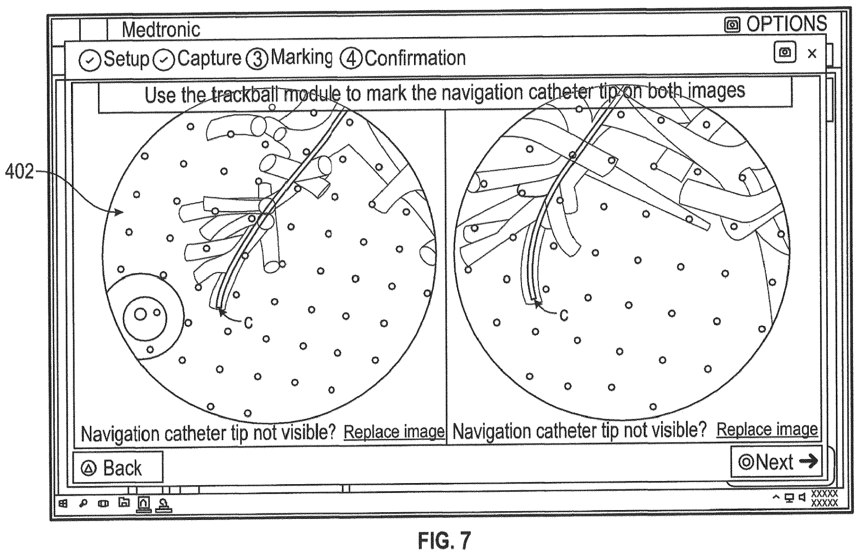

At step 310 a user-interface (FIG. 7) may be displayed on computing device 122 in which two of the images acquired during the fluoroscopic sweep from step 302 are presented and a clinician is asked to identify the position of the distal end of the catheter 106 in those images.

From the markings of two images, which though described here as manual, could also be automatically detected using a process similar to the probability mapping described above, an initial estimate for a static 3D catheter tip position in 3D space can be calculated. At step 312 the calculated position of the catheter 106 is projected on all of the remaining images of the fluoroscopic sweep acquired in step 302.

While the methods described herein relies on the marking of tip of the catheter 106 in two fluoroscopic images, the present disclosure is not so limited. In accordance with a further aspect of the disclosure the position of the catheter 106 can be determined based on marking of the tip of the catheter 106 in a single fluoroscopic image. This can be accomplished with reference to the detected position of the catheter 106 using the EMN system and sensor 104.

Because the spacing of the sphere markers 220 in the grid structure (FIG. 2) is known and spaced at a predefined distance from the antennae in the transmitter matt 120, the EM position of each sphere marker 230 in the field generated by the transmitter matt 120 is known. When considering a single image, and the position of the tip of the catheter 106, the catheter's vertical position (i.e., in the anterior-posterior direction) can be determined by comparing the EM position of the sphere markers to the detected position of the EM sensor 104 which should substantially correlate to the position of the tip of the catheter 106 along the AP axis in fluoroscopic imaging coordinates. The remaining two coordinates for the position of the tip of the catheter 106 can be resolved using a variety of processing techniques. By marking the tip of the catheter 106 in a frame, two values are provided that can be employed to generate two linear equations. These two linear equations can be solved to give the remaining two coordinates of the location of the tip of the catheter 106.

As with the probability mapping described above with respect to FIGs. 6A-6C, the 2D fluoroscopic images in which the position of the catheter 106 has been projected may be cropped at step 314, for example producing an image of 1/4^{th} or ½ the size of the original image. Of course, the full image or other size cropped images may also be processed by the next steps without departing from the scope of the disclosure. These cropped images define a region of interest and reduce the volume of data to be analyzed by subsequent steps.

At step 316, a trained model for catheter tip detection or some other appropriate learning software or algorithm, which is in communication with the computing device 122 accesses the 2D fluoroscopic images in which the position of the tip of the catheter 106 has been projected. The model may be a neural network that has been trained to identify a catheter tip at or above a certain confidence level. This is done by allowing the neural network to analyze images (e.g., from a fluoroscopic sweep) in which a catheter appears and allowing the neural network to perform image analysis to identify the location of the catheter tip. The actual location of the tip of the catheter 106 in each image or frame of the fluoroscopic sweep is known before being provided to the neural network for processing. A score is provided following each analysis of each frame by the neural network. Over time and training, the neural network becomes more adept at distinguishing the catheter 106 and particularly the tip of the catheter 106 as distinct from the tissues of the patient or other material the catheter 106 is in when the images are acquired. The result is a model or neural network that when used to analyze image identify the location of the tip of the catheter 106 with high confidence. Examples of neural networks that can be used to generate the model include a convolutional neural network or a fully connected network.

In order to improve the model or neural network, it must be trained to detect the position of the catheter 106. The suggested regression neural network is trained in a supervised manner. The training set consist of thousands of fluoroscopy 2D images with the compatible catheter tip coordinates marked manually. One method of training the neural network is to identify every frame of a fluoroscopic video as a main frame, and for each main frame identify at least one reference frame, and in some embodiments two reference frames. These reference frames may be sequentially immediately before and after the main frame, or at greater spacing (e.g., 10, 15, or 20 frames before and after). The reference frames assist in exploiting the temporal information in the fluoroscopic video to assist in estimating the coordinates of the tip of the catheter 106. There should only be small changes in position between the main frame and the reference frames, so a detection at some distance outside of an acceptable range will be determined to be a false positive detection by the neural network. By repeating the processing of images and detection of patterns which represent the catheter 106, the neural network is trained to detect the tip of the catheter. As noted above, the frames being analyzed may have been cropped prior to this analysis by the neural network. The neural network analyzes multiple frames may be processed in parallel, which assists in regularization of the process and provides more information to the neural network to further refine the training.

During the training, of the neural network a minimization of a loss function is employed. One such loss function is the comparison of the movement of the tip of the catheter 106 in successive frames. If the distance of movement exceeds an average movement between frames, then the score for that frame and its reference frames is reduced by the loss function. Heuristics can be employed to determine false detections. These false detections may occur when the tip of the catheter 106 is obscured in an image and cannot be easily detected. The false detections are a part of the training process, and as training continues these will be greatly reduced as the neural network learns the patterns of the catheter 106 in the images.

With the model or neural network having identified the tip of the catheter 106 in each frame, the 2D position of the tip catheter 106 in each fluoroscopic image is now known with even greater precision. This data can be used by the computing device at step 318 to update the pose estimation of the fluoroscopic imaging device 124 for each image acquired during the fluoroscopic sweep at step 304. In one embodiment, this can be achieved by repeating the pose estimation process for each frame employing the additional information of the catheter 106 position from the preceding iteration until all of the frames have been processed.

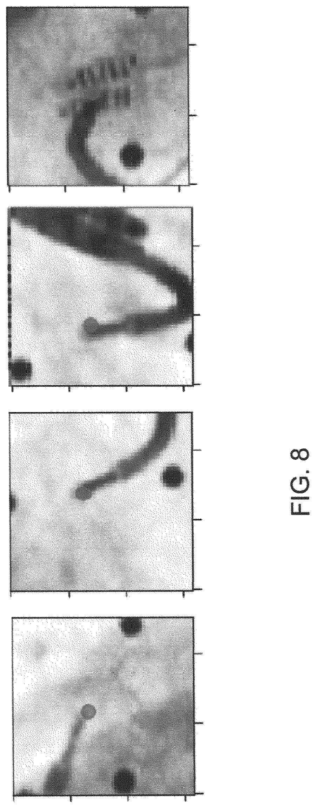

FIG. 8 depicts the results of a processing by the model or neural network identifying the tip of a catheter 106 in a series of images. Note that using this process, once the neural network is trained, it can achieve very accurate identification of the tip of the catheter 106 in a variety of positions and angles to the fluoroscopic imaging device 124. In one non-limiting example a Euclidian distance between the manual ground truth and the tip coordinates identified by the neural network was 0.31 mm with a standard deviation of 0.29 mm, following training of the neural network on 150 fluoroscopic videos (e.g., from sweep of step 302).

In instances where EMN system or another catheter location is being used employed, the detected 3D position of the tip of the catheter from that system may be combined with the detected 2D positions derived by the model or neural network to provide a more robust determination of the location of the tip of the catheter 106. In addition, such information can be employed to register the fluoroscopic images acquired at step 304 with a pre-operative image such as a CT scan with which a navigation plan has been developed.

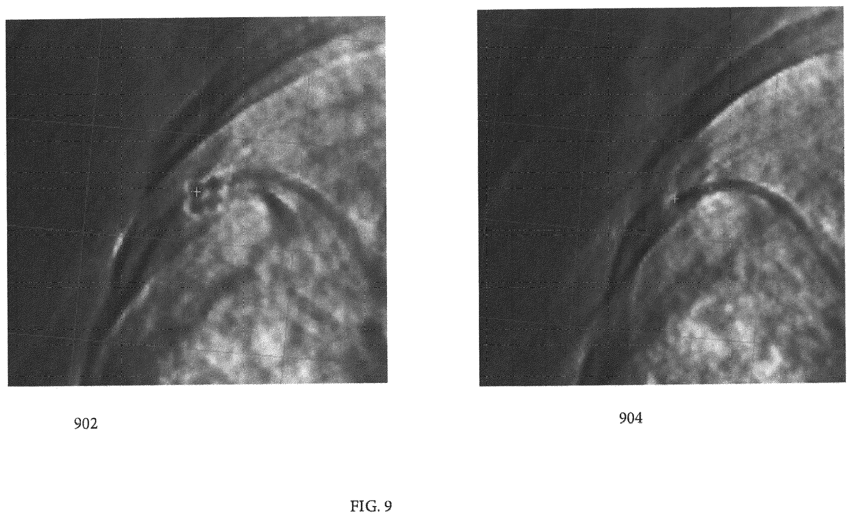

With an updated pose estimation of the imaging device 124, a 3D reconstruction of the fluoroscopic sweep can be generated by the computing device 122 at step 320. Because of the enhanced pose estimation provided by the use of the tip of the catheter 106 and the processing performed by the neural network the sharpness of the 3D reconstruction is greatly enhanced beyond traditional methods of fluoroscopic 3D reconstruction. An example of the heightened sharpness can be observed in FIG. 9, where following the 3D reconstruction an FCT image can be displayed at step 322. In FIG. 9 two FCT images are depicted, image 902 is an FCT image derived from pose estimation techniques without using the neural network and the methods described in FIG. 3. Image 904 depicts an FCT image achieved by utilizing the methods of FIG. 3. As can be seen, image 904 displays significantly greater sharpness of the catheter 106 as well as the soft tissues of the lung. This increase in sharpness allows for real time review of the FCT images to identify small lesions and ground glass lesions that are typically not observable in fluoroscopic images.

In order to improve the results of the method of FIG. 3 several post processing techniques may be employed. For example, the detection of the tip of the catheter 106 in each frame by the model or neural network may be given a confidence estimate. As a result, where there are frames in which the confidence estimate is low, the detection of the catheter 106 may be rejected. The position of the tip of the catheter 106 may be acquired from two frames in which detection has a high confidence, and then interpolated to find a better estimate of the position of the tip of the catheter 106 in the original frame. The confidence estimate may be the result of a low signal to noise ratio in a particular frame or the appearance of a major occlusion in the frame. For example, a comparison of the main portion of a frame with the median or average signal to noise ratio can reveal that the main portion of the frame is actually an occlusion and therefore should be rejected. Other methods of detecting occlusions or determining a confidence estimate for a given frame may be employed without departing from the scope of the disclosure. The frames used for the interpolation can be any frames and need not be similarly spaced from the frame with the low confidence of catheter tip detection. They may be the closest frames in which there is a high confidence of detection, or any pair of frames in which there was a high confidence of detection. Generally, however, the difference in position between the frames should be as small as practicable to achieve accurate interpolation of the position. Following the use of interpolation to overcome a smoothing algorithm may be employed to further refine the determination of the position of the tip of the catheter 106 in those frames in which there was a low confidence of detection such as those in which an occlusion was identified.

Reference is now made to FIG. 10, which is a schematic diagram of a system 1000 configured for use with the methods of the disclosure including the methods of FIG. 3. System 1000 may include a workstation 1001, and optionally connected to fluoroscopic imaging device 124 (FIG. 1). In some embodiments, workstation 1001 may be coupled with fluoroscope 1015, directly or indirectly, e.g., by wireless communication. Workstation 1001 may include a memory 1002, a processor 1004, a display 1006 and an input device 1010. Processor or hardware processor 1004 may include one or more hardware processors. Workstation 1001 may optionally include an output module 1012 and a network interface 1008. Memory 1002 may store an application 1018 and image data 1014. Application 1018 may include instructions executable by processor 1004 for executing the methods of the disclosure including the method of FIG. 3.

Application 1018 may further include a user interface 1016. Image data 1014 may include the CT scans, fluoroscopic images, the generated fluoroscopic 3D reconstructions and/or any other fluoroscopic image data and/or the generated one or more virtual fluoroscopy images. Processor 1004 may be coupled with memory 1002, display 1006, input device 1010, output module 1012, network interface 1008 and fluoroscope 1015. Workstation 1001 may be a stationary computing device, such as a personal computer, or a portable computing device such as a tablet computer. Workstation 1001 may embed a plurality of computer devices.

Memory 1002 may include any non-transitory computer-readable storage media for storing data and/or software including instructions that are executable by processor 1004 and which control the operation of workstation 1001 and, in some embodiments, may also control the operation of fluoroscope 1015. Fluoroscopic imaging device 124 may be used to capture a sequence of fluoroscopic images based on which the fluoroscopic 3D reconstruction is generated and to capture a live 2D fluoroscopic view according to this disclosure. In an embodiment, memory 1002 may include one or more storage devices such as solid-state storage devices, e.g., flash memory chips. Alternatively, or in addition to the one or more solid-state storage devices, memory 1002 may include one or more mass storage devices connected to the processor 1004 through a mass storage controller (not shown) and a communications bus (not shown).

Although the description of computer-readable media contained herein refers to solid-state storage, it should be appreciated by those skilled in the art that computer-readable storage media can be any available media that can be accessed by the processor 1004. That is, computer readable storage media may include non-transitory, volatile and non-volatile, removable and non-removable media implemented in any method or technology for storage of information such as computer-readable instructions, data structures, program modules or other data. For example, computer-readable storage media may include RAM, ROM, EPROM, EEPROM, flash memory or other solid-state memory technology, CD-ROM, DVD, Blu-Ray or other optical storage, magnetic cassettes, magnetic tape, magnetic disk storage or other magnetic storage devices, or any other medium which may be used to store the desired information, and which may be accessed by workstation 1001.

Application 1018 may, when executed by processor 1004, cause display 1006 to present user interface 1016. User interface 1016 may be configured to present to the user a single screen including a three-dimensional (3D) view of a 3D model of a target from the perspective of a tip of a medical device, a live two-dimensional (2D) fluoroscopic view showing the medical device, and a target mark, which corresponds to the 3D model of the target, overlaid on the live 2D fluoroscopic view as well as other images and screens described herein. User interface 1016 may be further configured to display the target mark in different colors depending on whether the medical device tip is aligned with the target in three dimensions.

Network interface 1008 may be configured to connect to a network such as a local area network (LAN) consisting of a wired network and/or a wireless network, a wide area network (WAN), a wireless mobile network, a Bluetooth network, and/or the Internet. Network interface 1008 may be used to connect between workstation 1001 and fluoroscope 1015. Network interface 1008 may be also used to receive image data 1014. Input device 1010 may be any device by which a user may interact with workstation 1001, such as, for example, a mouse, keyboard, foot pedal, touch screen, and/or voice interface. Output module 1012 may include any connectivity port or bus, such as, for example, parallel ports, serial ports, universal serial busses (USB), or any other similar connectivity port known to those skilled in the art.

While several aspects of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular aspects.

The invention may be described by reference to the following numbered paragraphs:-
1. A method for enhancing fluoroscopic computed tomography images comprising:
   acquiring a plurality of fluoroscopic images;
   determining an initial pose estimation of a fluoroscopic imaging device for each of the plurality of fluoroscopic images;
   receiving an indication of a catheter tip in at least one of the fluoroscopic images;
   projecting a position of the catheter tip in the remaining fluoroscopic images;
   analyzing the images with a model to identify a location of the catheter tip in the fluoroscopic images;
   updating the initial pose estimation based on the location of the catheter tip identified by the model; and
   generating a three-dimensional (3D) reconstruction of the plurality of fluoroscopic images utilizing the updated pose estimation.
2. The method of paragraph 1, further comprising displaying a fluoroscopic computed tomography image derived from the 3D reconstruction.
3. The method of paragraph 1, further comprising cropping the plurality of fluoroscopic images on which the position of the catheter tip was projected to define a region of interest.
4. The method of paragraph 3, further comprising determining a confidence estimate for the detection of the catheter tip in each cropped fluoroscopic image .
5. The method of paragraph 4, further comprising identifying two additional cropped fluoroscopic images located on either side, in the order in which the plurality of fluoroscopic images was acquired, of a cropped fluoroscopic image having a confidence estimate below a determined threshold.
6. The method of paragraph 5, wherein the two additional cropped fluoroscopic images have a confidence estimate higher than the determined threshold.
7. The method of paragraph 5, further comprising interpolating a position of the tip of the catheter in the cropped fluoroscopic image with a confidence estimate lower than the determined threshold from the position of the tip of the catheter in the two cropped fluoroscopic images with a confidence estimate higher than the determined threshold..
8. The method of paragraph 1, wherein the received indications of the catheter tip is automatically generated.
9. The method of paragraph 1, wherein the initial estimate of pose includes generating a probability map for each of the plurality of fluoroscopic images indicating a probability that each pixel of the fluoroscopic image belongs to a projection of a marker.
10. The method of paragraph 9, further comprises generating candidates for projection of the marker on the fluoroscopic image.
11. The method of paragraph 10, further comprising identifying the candidate with the highest probability of projection of the marker being the projection of the marker on the image based on the probability map.
12. A system for enhancing fluoroscopic computed tomography images comprising:
   a computing device in communication with a fluoroscopic imaging device and including a processor and a memory, the memory configured to store a plurality of fluoroscopic images and
   an application that when executed by the processor causes the processor to execute the steps of:
      determining an initial pose estimation of a fluoroscopic imaging device for each of the plurality of fluoroscopic images;
      receiving an indication of a catheter tip in at least one of the fluoroscopic images;
      projecting a position of the catheter tip in the remaining fluoroscopic images; and
      cropping the fluoroscopic images with the projected position of catheter tip to define a set of frames;
      a neural network in communication with the memory and configured to analyze the frames to identify a location of the catheter tip in the frames;
      the memory receiving the identified locations of the tip of the catheter in the frames from the neural network and the processor executing steps of the application of updating the initial pose estimation based on the location of the catheter tip identified by the neural network; and
      generating a three-dimensional (3D) reconstruction of the plurality of fluoroscopic images utilizing the updated pose estimation.
13. The system of paragraph 12, wherein the processor further executes steps of the application of displaying a fluoroscopic computed tomography image derived from the 3D reconstruction.
14. The system of paragraph 12, wherein the processor further executes steps of the application of generating a confidence estimate of detection of the tip of the catheter for each cropped fluoroscopic image.
15. The system of paragraph 14, wherein the processor further executes steps of the application of identifying two additional cropped fluoroscopic images located on either side, in the order in which the plurality of fluoroscopic images was acquired, of a cropped fluoroscopic image having a confidence estimate below a determined threshold.
16. The system of paragraph 15, wherein the two additional cropped fluoroscopic images have a confidence estimate higher than the determined threshold.
17. The system of paragraph 16, wherein the processor further executes steps of the application of interpolating a position of the tip of the catheter in the cropped fluoroscopic image with a confidence estimate lower than the determined threshold from the position of the tip of the catheter in the two cropped fluoroscopic images with a confidence estimate higher than the determined threshold.
18. The system of paragraph 17, wherein the initial estimate of pose includes generating a probability map for each of the plurality of fluoroscopic images indicating a probability that each pixel of the fluoroscopic image belongs to a projection of a marker.
19. The system of paragraph 18, further comprises generating candidates for projection of the marker on the fluoroscopic image and identifying the candidate with the highest probability of projection of the marker being the projection of the marker on the image based on the probability map.
20. A method for enhancing fluoroscopic computed tomography images comprising:
   acquiring a plurality of fluoroscopic images;
   determining an initial pose estimation of a fluoroscopic imaging device for each of the plurality of fluoroscopic images;
   receiving an indication of a catheter tip in at least two of the fluoroscopic images;
   projecting a position of the catheter tip in the remaining fluoroscopic images;
   cropping the plurality of fluoroscopic images on which the position of the catheter tip was projected to generate a plurality of frames;
   analyzing each frame to determine a location of the catheter tip in the frame;
   comparing the position of the catheter tip in each frame to a position of the catheter tip in at least two additional frames to confirm the determined location of the catheter tip in each frame;
   updating the initial pose estimation based on the confirmed location in each frame;
   generating a three-dimensional (3D) reconstruction of the plurality of fluoroscopic images utilizing the updated pose estimation; and
   displaying a fluoroscopic computed tomography image derived from the 3D reconstruction.

## Claims

1. A method for enhancing fluoroscopic computed tomography images comprising:
acquiring a plurality of fluoroscopic images;
determining an initial pose estimation of a fluoroscopic imaging device for each of the plurality of fluoroscopic images;
receiving an indication of a catheter tip in at least one of the fluoroscopic images;
projecting a position of the catheter tip in the remaining fluoroscopic images;
analyzing the images with a model to identify a location of the catheter tip in the fluoroscopic images;
updating the initial pose estimation based on the location of the catheter tip identified by the model; and
generating a three-dimensional (3D) reconstruction of the plurality of fluoroscopic images utilizing the updated pose estimation.

2. The method of claim 1, further comprising displaying a fluoroscopic computed tomography image derived from the 3D reconstruction.

3. The method of claim 1 or claim 2, further comprising cropping the plurality of fluoroscopic images on which the position of the catheter tip was projected to define a region of interest; preferably further comprising determining a confidence estimate for the detection of the catheter tip in each cropped fluoroscopic image .

4. The method of claim 3, further comprising identifying two additional cropped fluoroscopic images located on either side, in the order in which the plurality of fluoroscopic images was acquired, of a cropped fluoroscopic image having a confidence estimate below a determined threshold; preferably wherein the two additional cropped fluoroscopic images have a confidence estimate higher than the determined threshold.

5. The method of claim 4, further comprising interpolating a position of the tip of the catheter in the cropped fluoroscopic image with a confidence estimate lower than the determined threshold from the position of the tip of the catheter in the two cropped fluoroscopic images with a confidence estimate higher than the determined threshold..

6. The method of any preceding claim wherein the received indications of the catheter tip is automatically generated; and/or wherein the initial estimate of pose includes generating a probability map for each of the plurality of fluoroscopic images indicating a probability that each pixel of the fluoroscopic image belongs to a projection of a marker.

7. The method of claim 6, further comprises generating candidates for projection of the marker on the fluoroscopic image; preferably, further comprising identifying the candidate with the highest probability of projection of the marker being the projection of the marker on the image based on the probability map.

8. A system for enhancing fluoroscopic computed tomography images comprising:
a computing device in communication with a fluoroscopic imaging device and including a processor and a memory, the memory configured to store a plurality of fluoroscopic images and
an application that when executed by the processor causes the processor to execute the steps of:
determining an initial pose estimation of a fluoroscopic imaging device for each of the plurality of fluoroscopic images;
receiving an indication of a catheter tip in at least one of the fluoroscopic images;
projecting a position of the catheter tip in the remaining fluoroscopic images; and
cropping the fluoroscopic images with the projected position of catheter tip to define a set of frames;
a neural network in communication with the memory and configured to analyze the frames to identify a location of the catheter tip in the frames;
the memory receiving the identified locations of the tip of the catheter in the frames from the neural network and the processor executing steps of the application of updating the initial pose estimation based on the location of the catheter tip identified by the neural network; and
generating a three-dimensional (3D) reconstruction of the plurality of fluoroscopic images utilizing the updated pose estimation.

9. The system of claim 8, wherein the processor further executes steps of the application of displaying a fluoroscopic computed tomography image derived from the 3D reconstruction; preferably wherein the processor further executes steps of the application of generating a confidence estimate of detection of the tip of the catheter for each cropped fluoroscopic image.

10. The system of claim 9, wherein the processor further executes steps of the application of identifying two additional cropped fluoroscopic images located on either side, in the order in which the plurality of fluoroscopic images was acquired, of a cropped fluoroscopic image having a confidence estimate below a determined threshold; preferably wherein the two additional cropped fluoroscopic images have a confidence estimate higher than the determined threshold.

11. The system of claim 10, wherein the processor further executes steps of the application of interpolating a position of the tip of the catheter in the cropped fluoroscopic image with a confidence estimate lower than the determined threshold from the position of the tip of the catheter in the two cropped fluoroscopic images with a confidence estimate higher than the determined threshold.

12. The system of claim 11, wherein the initial estimate of pose includes generating a probability map for each of the plurality of fluoroscopic images indicating a probability that each pixel of the fluoroscopic image belongs to a projection of a marker.

13. The system of claim 12, further comprises generating candidates for projection of the marker on the fluoroscopic image and identifying the candidate with the highest probability of projection of the marker being the projection of the marker on the image based on the probability map.

14. A method for enhancing fluoroscopic computed tomography images comprising:
acquiring a plurality of fluoroscopic images;
determining an initial pose estimation of a fluoroscopic imaging device for each of the plurality of fluoroscopic images;
receiving an indication of a catheter tip in at least two of the fluoroscopic images;
projecting a position of the catheter tip in the remaining fluoroscopic images;
cropping the plurality of fluoroscopic images on which the position of the catheter tip was projected to generate a plurality of frames;
analyzing each frame to determine a location of the catheter tip in the frame;
comparing the position of the catheter tip in each frame to a position of the catheter tip in at least two additional frames to confirm the determined location of the catheter tip in each frame;
updating the initial pose estimation based on the confirmed location in each frame;
generating a three-dimensional (3D) reconstruction of the plurality of fluoroscopic images utilizing the updated pose estimation; and
displaying a fluoroscopic computed tomography image derived from the 3D reconstruction.
